# EUROPÄISCHE PATENTANMELDUNG

(11) **EP 0 602 317 A2**
(43) Veröffentlichungstag der Anmeldung: **22.06.1994**
(21) Anmeldenummer: 93112490.3
(22) Anmeldetag: 04.08.1993
(51) Int. Cl.: A61F 13/06, A61F 5/01

(54) **Bandage für das Kniegelenk**

(30) Priorität: 05.11.1992 DE 4237389
(71) Anmelder: Beiersdorf Aktiengesellschaft, D-20245 Hamburg (DE)
(72) Erfinder: Brandt, Dieter, D-40545 Düsseldorf (DE); Szlema, Ingeborg M. A., D-47906 Kempen (DE)
(74) Vertreter: Richter, Werdermann & Gerbaulet

(57) **Zusammenfassung**

Die Bandage (10) für Überlastungserscheinungen, femoropatellare Schmerzsyndrome und das Patellaspitzensyndrom aus einem elastischen Bandagenstoff in Schlauchform mit einem rundherum laufenden Einsatz (30) aus einem Wellengestrick (40) weist in dem vorderen Bandagenteil (12) eine im Bereich über der Kniescheibe bei angelegter Bandage liegende, nach oben offene und die Quadrizeps-Sehne freilassende Pelotte (50) sowie zusätzlich eine die Pelotte (50) stabilisierende Spange (70) auf.

## Beschreibung

Die Erfindung betrifft eine Bandage für das Kniegelenk aus elastischem Bandagenstoff in Schlauchform mit einem vorderen Bandagenteil und einem rückwärtigen Bandagenteil und mit gegebenenfalls im rückwärtigen Bandagenteil eingearbeiteten, längsverlaufenden Federstäben.

Das Knie ist das verletzungsgefährdeste und am häufigsten von Arthrosen betroffene Gelenk des Körpers, da es instabil ist und das volle Körpergewicht trägt. Häufig sind auch muskuläre Disbalancen Ursache für Schmerzen an der vorderen Kniescheibe. Vor allem bei Personen mit sitzenden Berufen ist die ischiokrurale Muskulatur meist verkürzt und zieht die Gelenkkapsel nach hinten, wodurch sich der Anpreßdruck der Kniescheibe unphysiologisch erhöht. Sportverletzungen des Kniegelenkes entstehen vor allem beim Fußballspielen und beim Skilaufen durch plötzliche Drehungen und Belastungen des Unterschenkels. Affektionen des Knies haben meist örtliche Schmerzen zur Folge. Die Komplexität des Gelenks erschwert die genaue Diagnose. Deshalb kommt es häufig vor, daß Patienten nicht die richtige Anfangsbehandlung erhalten, was zu späteren degenerativen Erkrankungen führen kann.

Zur Versorgung von Knieverletzungen steht eine Vielfalt therapeutischer Hilfsmittel zur Ruhigstellung oder Bewegungseinschränkung sowie zur Stützung und Entlastung des Kniegelenkes zur Verfügung. Auch der Bereich der Aktiv-Bandagen ist mit einer Vielzahl von Produkten besetzt, wie sie bei keinem anderen Körperteil zu finden ist. So ist durch die DE-A- 34 16 231 eine Kniebandage zum Schlüpfen aus elastischem Material mit gegebenenfalls eingearbeiteten, längsverlaufenden Federn zur Verstärkung der Medial- und Lateralseite des Kniegelenkes, eine Aussparung für die Patella und eine diese Aussparung umgebende Polsterung bekannt, wobei die die Aussparung umgebende Polsterung derart ausgeführt ist, daß sich ein Anpreßdruck auf das Kniescheibenband ergibt, der das femoro-patellare Gleitlager entlastet. Die Polsterung umgibt dabei die Aussparung U-förmig, wobei die U-Form nach oben geöffnet ist. Die Polsterung stellt ein elastisches, in die Kniebandage eingearbeitetes U-Element dar, das die Aussparung ausschließlich seitlich und von unten umgibt. Dieses Element besteht aus Silikonmaterial oder Gummi. Des weiteren ist das U-Element als Wulst mit konvexer Seite zum Körper ausgebildet. Wesentlich ist jedoch bei dieser Kniebandage, daß das Hauptvolumen der Polsterung bzw. des Wulstes über dem Kniescheibenband angeordnet ist. Neben einem angenehmen Tragegefühl soll eine optimale Entlastung des Kniegelenkes bei gleichzeitiger Führung desselben erreicht werden, um hierdurch im Falle von Verletzungen, Reizungen oder anderen pathologischen Zuständen einen möglichst kurzen und schonenden Heilungsverlauf zu erreichen. Allerdings erfolgt bei dieser Kniebandage keine Druckentlastung auf das Kniescheibenband, denn es soll ja gerade ein Anpreßdruck auf das Kniescheibenband erreicht werden. Die Kniegelenkbandage nach DE-U- 90 04 974 besteht aus einem sich konisch nach unten verjüngenden Kniestrumpf aus einem elastischen Material, wie einem Gummigewebe. Der Kniestrumpf trägt auf seiner Vorderseite im Bereich der Kniescheibe zwei von oben sich nach unten erstreckende Halterungen, die die Kniescheibe seitlich auf ihrer Unterseite anliegend abstützen. Diese Kniegelenkbandage soll eine höhenmäßige Veränderung der Kniescheibe zu den Muskeln erlauben, jedoch die Kniescheibe gegenüber der Gleitfläche in angehobenem Zustand durch eine unterseitige Abstützung der Kniescheibe halten.

Die DE-A- 39 91 334 beschreibt eine elastische Kniegelenkbandage in Schlauchform mit einer elastischen, die Kniescheibe in einer Aussparung umfassenden Profileinlage. In die Profileinlage ist in festem Verbund mit dieser ein biegsames, nicht dehnbares Spannglied eingelagert, das die Bereiche der Profileinlage benachbart zu den Kniescheibenpolen wadenbeinseitig im Bogen um die Kniescheibe derart verbindet, daß bei Vergrößerung der Entfernung dieser Bereiche beim Beugen des Kniegelenkes der Abstand des Bogens von der Verbindungslinie der Kniescheibenpole verringert wird und der betreffende Rand der Aussparung der Profileinlage auf die benachbarte Seite der Kniescheibe diese medial verschiebend und zentrierend drückt. Mit dieser Kniegelenkbandage soll auf eine Kniescheibe, die aus ihrer Idealposition entweder krankhaft oder als sehr häufige Normvariante wadenbeinseitig verschoben ist, beim Beugen des Kniegelenkes dynamisch eingewirkt werden, um dabei die Kniescheibenlage zu korrigieren. Beim Beugen des Kniegelenkes wird jedoch die bei dieser Kniegelenkbandage verwendete Spange auseinandergezogen mit der Folge, daß sich die Spange aufrichtet und die gewünschte zentrierende Wirkung nicht voll erreicht wird.

Der Erfindung liegt die Aufgabe zugrunde, eine Bandage für das Kniegelenk, im nachfolgenden als Genu-Kniegelenkbandage, und eine darauf aufbauende, nachfolgend als Patella-Kniegelenkbandage bezeichnete Kniegelenkbandage für den Einsatz bei einer Lateralisationstendenz der Patella zu schaffen, mit der folgendes erreicht werden soll:
- Unterstützung der Physiotherapie. Wichtiger Bestandteil der krankengymnastischen Behandlung ist die Dehnung der ischiokruralen Muskeln.
- Entlastung des Kniegelenkes und der Patella.
- Herbeiführung einer raschen Abschwellung, Schmerzlinderung und Funktionsverbesserung.
- Ausübung einer intermittierenden Kompression und einer Massage auf die Gelenkweichteile.
- Vermeidung eines unerwünschten Druckes auf die Patella und den darunterliegenden Knorpelbelag.
- Verringerung des Anpreßdruckes auf die Patella.
- Erhaltung der physiologischen Gleitfähigkeit der Patella.
- Entlastung des Ansatzes der ischiokruralen Muskeln.
- Vermeidung eines unerwünschten Druckes auf die Nerven und Gefäße, die in der Kniekehle verlaufen.
- Vermeidung von negativen Auswirkungen auf die Muskelaktivität.
- Vermeidung einer Faltenbildung der Bandage in der Kniekehle.
- Keine Beeinträchtigung der Funktion der Quadrizeps-Sehne, die Patella in ihre richtige Position zu ziehen,
wobei die Patella-Kniegelenkbandage darüber hinaus folgende Eigenschaften haben soll:
- Seitliche Führung der Patella zur Verringerung der Lateralabweichung.

Gelöst wird diese Aufgabe bei einer Bandage für Kniegelenke durch die in den Ansprüchen 1,2,3 und 4 angegebenen Merkmale.

Eine Genu-Kniegelenkbandage besteht nach Anspruch 1 aus einem anatomisch geformten Schlauchkörper aus einem Gewebe oder Gestrick mit einem rundumlaufenden Einsatz aus einem hochelastischen Wellengestrick, wobei über den das rückwärtige Bandagenteil mit dem vorderen Bandagenteil verbindenden Nähten Federstäbe in das Schlauchkörpermaterial eingearbeitet sind. In dem vorderen Bandagenteil ist eine im Bereich über der Kniescheibe bei angelegter Bandage liegende erste, die Quadrizeps-Sehne freilassende Pelotte aus einem weichen oder weich-elastischen Material angeordnet. In das rückwärtige Bandagenteil ist nach den Ansprüchen 3 und 4 eine zweite oder eine zweite und eine dritte, den Ansatz der ischiokruralen Muskulatur beaufschlagende Pelotte aus einem weichen oder weich-elastischen Material eingesetzt.

Die Patella-Kniegelenkbandage ist entsprechend der Genu-Kniegelenkbandage ausgebildet; sie weist nach Anspruch 2 zusätzlich in ihrem vorderen Bandagenteil mindestens eine, die erste Pelotte stabilisierende, in Bandagenlängsrichtung verlaufende Spange auf, die integrierter Bestandteil der ersten Pelotte oder seitlich zu dieser liegend ist. Diese Spange ist elastisch und ist bevorzugterweise in Längsrichtung unelastisch.

Mit einer derart ausgebildeten Bandage für das Kniegelenk lassen sich folgende Indikationen am Knie wirkungsvoll behandeln.
- Reizzustände und Überlastungserscheinungen des Kniegelenks.
- Distorsionen und Kontusionen.
- Gelenkergüsse und Schwellungen bei Arthrose und Arthritis.
- Myotendopathien.
- Bänderschwäche.
- Patellaspitzensydrom.
- Luxationsneigung der Patella.
- Lateralisationstendenz der Patella.
- Chondropathia patellae.

Der Indikationsrahmen "schmerzhafte Reizzustände des Kniegelenks" ist sehr weit gefaßt und reicht von Weichteilschwellungen nach Sport- oder Arbeitsunfällen über chronische Beschwerden bis zu femoropatellaren Dysplasien mit Lateralisationstendenz. Letztere erfordern Konstruktionsmerkmale einer Kniebandage, die über die zur Therapie allgemeiner Kniebeschwerden nötigen Eigenschaften hinausgehen. Alle Wirkfaktoren in ein Produkt einzubauen, würde sich restriktiv auf den Indikationsbereich auswirken: Die Bandage wird zur Spezialbandage und würde z.B. bei unspezifischen Knieschmerzen eine Überversorgung darstellen. Deshalb ist es vorteilhaft, wenn zwei Bandagen eingesetzt werden, nämlich die Genu-Kniegelenkbandage als allgemeines Basis-Produkt für Überlastungserscheinungen, femoropatellare Schmerzsyndrome und für das Patellaspitzensyndrom, während die Patella-Kniegelenkbandage das auf dieser Genu-Kniegelenkbandage aufbauende Produkt darstellt und bei Lateralisationstendenz und chondropathischen Beschwerden eingesetzt wird. Beide Bandagen, die Genu-Kniegelenkbandage und die Patella-Kniegelenkbandage bauen aufeinander auf.

Deshalb sind die Wirkungsweisen und Vorteile für beide Bandagen weitgehend identisch, wobei für die Patella-Kniegelenkbandage außerdem noch weitere Vorteile hinzu kommen.

Mit der Genu- und Patella-Kniegelenkbandage wird die Physiotherapie unterstützt und das Kniegelenk und die Patella entlastet. Eine rasche Abschwellung, Schmerzlinderung und Funktionsverbesserung wird herbeigeführt. Darüber hinaus wird eine intermittierende Kompression auf die Gelenkweichteile und eine tiefenwirksame Massage ausgeübt. Ein unerwünschter Druck auf die Patella und den darunterliegenden Knorpelbelag wird vermieden. Der Anpreßdruck der Patella wird verringert, wobei deren physiologische Gleitfähigkeit erhalten wird. Der Ansatz der ischiokruralen Muskeln wird entlastet. Ein unerwünschter Druck auf die Nerven und Gefäße, die in der Kniekehle verlaufen, wird vermieden. Es ergeben sich keine negativen Auswirkungen auf die Muskelaktivität. Die Bandage schnürt nicht und ruft keine zirkulatorischen Schwierigkeiten hervor. Eine Faltenbildung der Bandage in der Kniekehle bei einer Kniebeugung wird minimiert. Die Bandage rutscht nicht und auch bei Bewegung wird ein kontinuierlich guter Sitz beibehalten. Mit der Bandage wird ein hoher Tragekomfort erreicht, da sie nicht scheuert und hautfreundlich ist. Außerdem hat die Kniegelenkbandage den Vorteil, daß die Funktion der Quadrizeps-Sehne, die Patella in ihre richtige Position zu ziehen, nicht beeinträchtigt wird. Außerdem erfolgt bei der Patella-Kniegelenkbandage eine seitliche Führung der Patella, wobei die Lateralabweichung verringert wird.

Die Genu-Kniegelenkbandage ist eine dreidimensional anatomisch formgestrickte Zweizug-Bandage, die vom Unterschenkel bis zum Oberschenkel reicht und eine funktionale leichte Beugestellung von etwa 10^{o} besitzt und mit Druckminderungsrändern ausgestattet ist. Die Bandage selbst besteht aus zwei Teilen, wobei das vordere Bandagenteil 2/3 des Beinumfanges umspannt. Über dem Kniegelenk befindet sich ein um das ganze Bein reichender Einsatz aus hochelastischem Wellengestrick. Das rückwärtige Bandagenteil wird mit vorgespannter Welle des Wellengestricks eingenäht.

Über den seitlichen Nähten in Taschen eingearbeitete Federstäbe halten die Bandage gespannt. Das Wellengestrick ist über der Patella geschlossen. Im Bereich über der Kniescheibe befindet sich eine bevorzugterweise hufeisenförmig ausgebildete erste Pelotte. Die nach oben geöffnete Pelotte läßt die Quadrizepssehne frei. Im unteren Teil ist das Profil der Pelotte dem Schienbeinkopf und dem Verlauf des Ligamentum patellae angepaßt. Diese im vorderen Bandagenteil angeordnete Pelotte enthält bevorzugterweise einen knopfförmigen Friktionskern aus einem Material, welches gegenüber dem Material der Pelotte einen höheren Härtegrad aufweist. Dieser Friktionskern ist infrapatellar angeordnet. In das rückwärtige Bandagenteil ist eine Pelotte oder sind zwei Pelotten eingenäht, die ellipsenförmig ausgebildet sind oder die andere geometrische Formen aufweisen und die den Ansatz der ischiokruralen Muskulatur beaufschlagen. Diese beiden Pelotten sind etwa 1 cm oberhalb des Kniegelenkspalts unmittelbar an den seitlichen Nähten plaziert, vermittels der das vordere Bandagenteil mit dem rückwärtigen Bandagenteil verbunden ist.

Auch die Patella-Kniegelenkbandage ist eine dreidimensional anatomisch formgestrickte Zweizug-Bandage für das Kniegelenk. Die Bandage reicht vom Unterschenkel bis zum Oberschenkel und hat eine funktionelle leichte Beugestellung von etwa 10^{o} und ist mit Druckminderungsrändern ausgestattet. Die Bandage besteht ebenfalls aus zwei Teilen, wobei das Vorderteil 2/3 des Beinumfangs umspannt. Über dem Kniegelenk befindet sich ein um das ganze Bein reichender Einsatz aus hochelastischem Wellengestrick. Das rückwärtige Bandagenteil wird mit vorgespannter Welle eingenäht. Über den seitlichen Nähten in Taschen eingearbeitete Federstäbe halten die Bandage gespannt. Das Gestrick ist über der Patella geschlossen. Im Bereich über der Kniescheibe befindet sich eine bevorzugterweise hufeisenförmige erste Pelotte. Die nach oben geöffnete Pelotte läßt die Quadrizeps-Sehne frei. Im unteren Teil ist das Profil der Pelotte dem Schienbeinkopf und dem Verlauf des Ligamentum patellae angepaßt. Die bei der Patella-Bandage verwendete Pelotte kann einen knopfförmigen Friktionskern enthalten, der aus einem Material besteht, dessen Härtegrad höher ist als der Härtegrad des Materials, aus dem die Pelotte hergestellt ist. Dieser Friktionskern ist dann infrapatellar angeordnet. Außerdem kann die Pelotte der Patella-Kniegelenkbandage eine Komponente mit unterschiedlichen Dehnungseigenschaften aufweisen. Die erste Pelotte ist seitlich mit einem fächerartigen Streifen bzw. mit einer Spange verbunden, die bevorzugterweise aus einem federnd-elastischen Material besteht, jedoch keine Längselastizität aufweist. Wenn es bei der Bewegung zwischen Streckung und Beugung zu einer Verlängerung der elastischen Elemente in der Bandage kommt, zieht sich dieser Streifen bzw. die Spange gerade und übt eine zentrierende Kraft auf die Pelotte aus. In das rückwärtige Bandagenteil der Patella-Kniegelenkbandage können zwei ellipsenförmige Pelotten eingenäht sein, die den Ansatz der ischiokruralen Muskulatur beaufschlagen. Diese beiden Pelotten sind etwa 1 cm oberhalb der Kniegelenkspalts unmittelbar an den seitlichen Nähten plaziert.

Vorteilhafte Ausgestaltungen der Erfindung sind in den Unteransprüchen gekennzeichnet.

Besonders vorteilhaft ist die Ausgestaltung des Wellengestrickes, aus dem der Einsatz besteht, der im Beugebereich der Bandage angeordnet ist. Dieses Wellengestrick besitzt zumindest auf einer Seite ein Relief in Form einer Wellenstruktur. Diese Wellenstruktur wird durch eine darunterliegende Fadenanordnung aus höherelastischem Garn bzw. Faden elastisch vorgespannt und stabilisiert, wodurch die Deckstruktur im entspannten Zustand des Wellengestricks wellenartig, vorzugsweise halbwellenartig,ausbaucht. Wenn dementsprechend diese Flächenstruktur bei einer Bandage eingesetzt und senkrecht zur Ausrichtung der Querwellen beansprucht wird, was beispielsweise beim Beugen des bandagierten Gelenkes der Fall ist, so werden zunächst lediglich die Querwellen flacher bzw. glatt gezogen , ohne daß eine Streckung bzw. Reckung der Deckstruktur erfolgt. Überdehnungen der Deckstruktur können auf diese Art und Weise zuverlässig ausgeschlossen werden, wodurch gleichzeitig das Auftreten von Falten, und zwar selbst nach längerem Gebrauch der Bandage, im innenliegenden Gelenkbeugebereich vermieden wird. Das Wellengestrick eignet sich deshalb insbesondere zur Verwendung in Bandagen, die für die Gelenke mit großem Bewegungsfreiraum bzw. Beugewinkel eingesetzt werden, wie das beispielsweise bei Kniegelenkbandagen der Fall ist. Besonders vorteilhaft ist dabei die Ausgestaltung, nach der das Wellengestrick eine zumindest auf einer Seite ausgebildete, im wesentlichen senkrecht zur Hauptdehnungsrichtung ausgerichtete Wellenstruktur einer Deckstruktur aufweist, wobei diese Wellenstruktur durch eine eingearbeitete oder unter dieser Deckstruktur liegende elastische Fadenanordnung elastisch vorgespannt und stabilisiert ist, die in vorbestimmten Abständen mit der Deckstruktur verbunden ist und derart geformt ist, daß die Anzahl der Querwellen dort am größtem ist, wo aufgrund der Gelenkbeugung der größte Dehnungsweg auftritt.

Ein derartiges Wellengestrick erlaubt somit trotz verhältnismäßig hohen Dehnungsvermögens die Verwendung von verhältnismäßig unelastischem Garn für die Deckstruktur, wodurch die Voraussetzung für eine wirtschaftliche Herstellung des Wellengestricks geschaffen wird. Des weiteren besteht bei dem Wellengestrick keine Gefahr von Überdehnungen des eingesetzten Garns, da eine große Grund-Elastizität vorhanden ist.

Ausführungsbeispiele der Erfindung werden nachstehend anhand der Zeichnung näher erläutert. Es zeigen
Fig. 1 in einer Ansicht von vorn eine Genu-Kniegelenkbandage mit einer U-förmigen Pelotte mit in deren rückwärtigen Bandagenteil angeordneten Federstäben und zwei ellipsenförmigen Pelotten,
Fig. 2 eine Rückansicht der Genu-Kniegelenkbandage,
Fig. 3 in einer Ansicht von vorn eine Patella-Kniegelenkbandage mit einer U-förmigen Pelotte und mit einer in einem Abstand von dieser seitlich angeordneten Pelottenzentrierungsspange und mit im rückwärtigen Bandagenteil angeordneten Federstäben und zwei ellipsenförmigen Pelotten,
Fig. 4 eine Rückansicht der Patella-Kniegelenkbandage,
Fig. 5 in einer Ansicht von vorn eine weitere Ausführungsform der Patella-Kniegelenkbandage mit einer U-förmigen Pelotte und mit einer seitlich von dieser angeordneten Pelottenzentrierungsspange,
Fig. 6 in einer Ansicht von vorn eine weitere Ausführungsform der Patella-Kniegelenkbandage mit einer U-förmigen Pelotte und mit einer in deren einen Pelottenschenkel integrierten Pelottenzentrierungsspange,
Fig. 7 in einer Ansicht von vorn die U-förmige Pelotte mit einer in deren einen Pelottenschenkel integrierten Pelottenzentrierungsspange,
Fig. 8 in einer Ansicht von vorn die U-förmige Pelotte mit einer an deren einen Pelottenschenkel angeformten Pelottenzentrierungsspange,
Fig. 9 in einer Ansicht von vorn eine Genu-Kniegelenkbandage mit einer U-förmigen, einen Friktionskern aufweisenden Pelotte,
Fig. 10 in einer Ansicht von vorn eine einen Friktionskern aufweisende U-förmige Pelotte,
Fig. 11 in einer Ansicht von vorn die U-förmige Pelotte,
Fig. 12 einen senkrechten Schnitt gemäß Linie XII-XII in Fig. 11,
Fig. 13 einen senkrechten Schnitt gemäß Linie XIII-XIII in Fig. 11,
Fig. 14 in einer Ansicht von vorn eine ellipsenförmige Pelotte,
Fig. 15 einen senkrechten Schnitt gemäß Linie XV-XV in Fig. 14,
Fig. 16 in einer Ansicht von vorn zwei über einen Steg miteinander verbundene ellipsenförmige Pelotten,
Fig. 17 einen senkrechten Schnitt gemäß Linie XVII-XVII in Fig. 16,
Fig. 18 in einer Ansicht von vorn eine U-förmige Pelotte mit seitlichen, über Stege mit der Pelotte verbundenen ellipsenförmigen Pelotten,
Fig. 19 einen senkrechten Schnitt gemäß Linie XIX-XIX in Fig. 18,
Fig. 20 in einer Ansicht von vorn eine Patella-Kniegelenkbandage mit einer U-förmigen Pelotte und mit zwei seitlich zu dieser angeordneten Pelottenzentrierungsspangen,
Fig. 21 in einer schematischen Ansicht von vorn einen in der Bandage rundum angeordneten Einsatz aus einem hochelastischen Wellengestrick,
Fig. 22 in einer Rückansicht den Einsatz aus dem hochelastischen Wellengestrick,
Fig. 23 in einer schematischen Darstellung die Ausbildung des hochelastischen Wellengestricks in dem vorderen Bandagenteil und in dem rückwärtigen Bandagenteil der Kniegelenkbandage,
Fig. 24 einen vergrößerten Schnitt durch den Einsatz aus dem Wellengestrick gemäß Linie XXIV-XXIV in Fig. 23,
Fig. 25 in einer schematisch vereinfachten Darstellung das Prinzip der Verformung eines aus dem Wellengestrick bestehenden Flächengebildes,
Fig. 26 Darstellungen von Strickreihen zur Verdeutlichung einer ersten Ausführungsform eines Verfahrens zur Herstellung des Wellengestricks,
Fig. 27 in einer der Fig. 26 ähnlichen Darstellung ein weiteres Strickmuster für das Wellengestrick,
Fig. 28 in einer Seitenansicht einen in der U-förmigen Pelotte angeordneten Friktionskern,
Fig. 29 eine Ansicht auf das menschliche Kniegelenk und
Fig. 30 in einem Querschnitt die rechte Tibia.

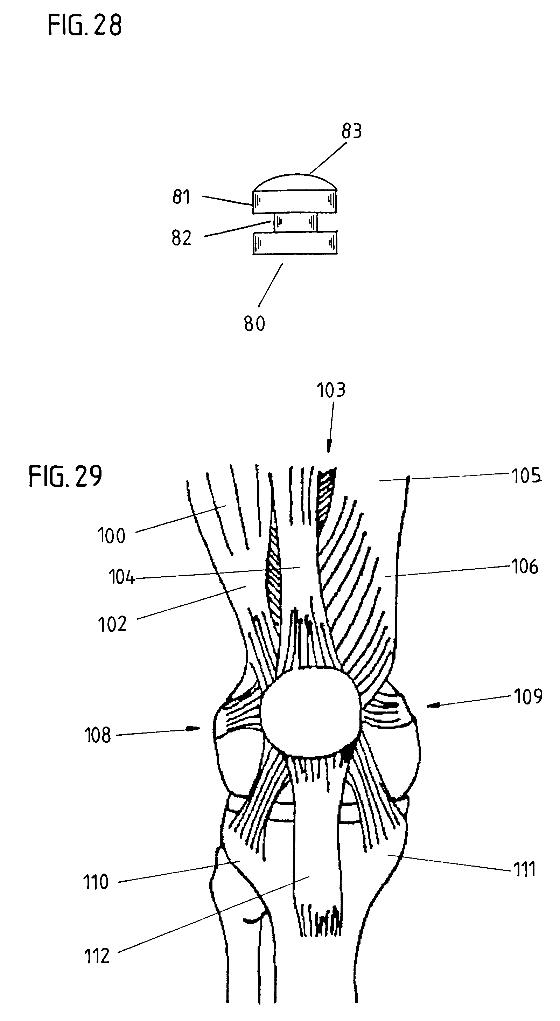

Das in der Mitte der Bewegungskette "untere Extremität" gelegene,in Fig. 29 wiedergegebene Kniegelenk ist das größte Gelenk des menschlichen Körpers. Der obere Gelenkkörper ist eine in der Mitte eingekerbte Rolle. Ihr steht eine nur vom Schienbein gebildete, sehr flache Gelenkfläche gegenüber. Die Menisken, zwei auf der Schienbeingelenkfläche liegende Knorpelplatten, stellen die Pfannen dar, in denen sich die Rolle des oberen Gelenkörpers scharnierartig bewegen kann. Eine knöchernde Führung des Kniegelenkes ist nicht vorhanden. Diese Funktion wird von anderen Strukturen übernommen. Die kräftigen Seitenbänder dienen als Führungsschienen für die Scharnierbewegung. Das Durchrutschen der gelenkbildenden Knochen nach hinten oder vorn verhindern die kurzen, im inneren des Gelenkes liegenden Kreuzbänder. Vor dem Gelenk liegt, in eine Sehne eingebettet, die Kniescheibe. Sie ist der größte freie Knochen und dient der Verbesserung der Zugwirkung des Unterschenkelstreckers. Die Patella-Sehne ist sehr empfindlich.

Bei dem in Fig. 29 dargestellten Kniegelenk ist mit 100 der Musculus vastus lateralis, mit 102 die Tendo musculi vasti lateralis, mit 103 der Musculus vastus intermedius, mit 104 der Musculus recto femoris, mit 105 der Musculus vastus medialis, mit 106 der Musculus vastus medialis obliquus, mit 107 die Patella, mit 108 das Ligamentum patellofemorale laterale, mit 109 das Ligamentum patellofemorale mediale, mit 110 das Ligamentum patellotibiale laterale, mit 111 das Ligamentum patellotibiale mediale und mit 112 das Ligamentum patellae bezeichnet.

Die Stabilisatoren des Kniegelenks werden in vier Funktionseinheiten dabei eingeteilt: Die Stabilisatoren des medialen und des lateralen Komplexes sowie die dorsalen und ventralen Strukturen, wobei zwischen statischen und dynamischen Stabilisatoren unterschieden wird.

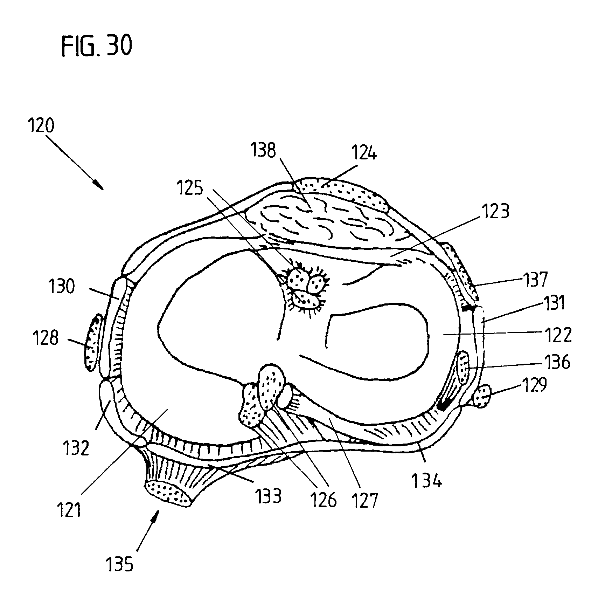

Fig. 30 zeigt die rechte Tibia 120 in einem Querschnitt. Hier sind mit 121 der Innenmeniskus, mit 122 der Außenmeniskus, mit 123 das Ligamentum genus transversum, mit 124 das Ligamentum patellae, mit 125 das Ligamentum cruciatum anterius, mit 126 das Ligamentum cruciatum posterius, mit 127 das Ligamentum meniscofemorale posterius, mit 128 das Ligamentum collaterale mediale, mit 129 das Ligamentum collaterale laterale, mit 130 das mediale Kapselband, mit 131 das laterale Kapselband, mit 132 das Ligamentum obliquum posterius, mit 133 das Ligamentum popliteum obliquum, mit 124 das Ligamentum popliteum arcuatum, mit 135 der Musculus semimembranosus, mit 136 der Musculus popliteus, mit 137 der Tractus iliotibialis und mit 138 der Hoffasche Fettkörper bezeichnet.

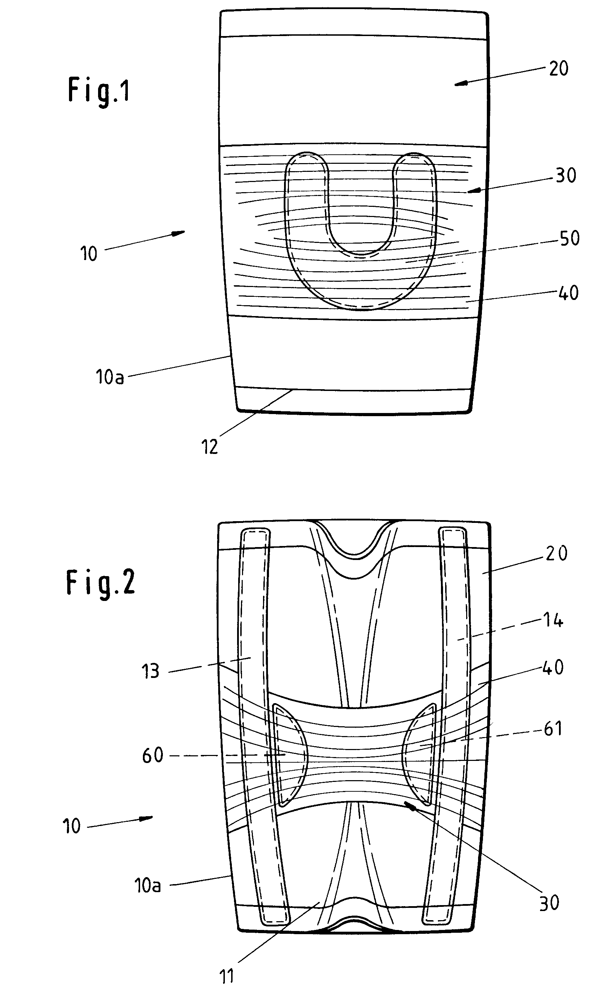

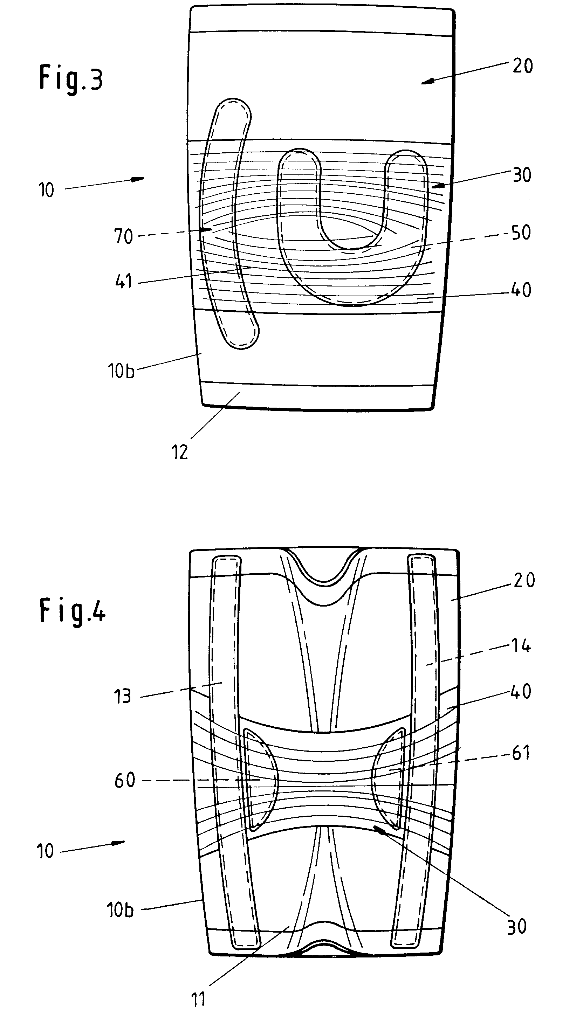

Die in der Zeichnung dargestellte Bandage 10 für ein Kniegelenk besteht aus einem anatomisch geformten Schlauchkörper 20 aus einem Gewebe oder Gestrick mit einem rundumlaufenden Einsatz 30 aus einem hochelastischen Wellengestrick 40. Über den das rückwärtige Bandagenteil 11 mit dem vorderen Bandagenteil 12 verbindenden Nähten sind Federstäbe 13,14 in das Schlauchkörpermaterial eingearbeitet, die entsprechend der anatomischen Form des Schlauchkörpers 20 bogenförmig verlaufend ausgebildet sind, wie dies in den Fig. 2 und 4 dargestellt ist. Diese Federstäbe 13,14 sind gerade verlaufend ausgebildet; sie werden erst hinterher den bogenförmigen Verlauf erhalten, wenn die Bandage ausgebildet wird.

In dem vorderen Bandangenteil 12 der Bandage 10 ist eine erste, nach oben offene und die Quatrizeps-Sehne freilassende Pelotte 50 aus einem weichen oder weich-elastischen Material angeordnet, wobei diese Pelotte 50 bei angelegter Bandage im Bereich über der Kniescheibe sitzt. Diese Pelotte 50 ist U-förmig ausgebildet und wird gebildet von den Schenkeln 51,52 und dem die Schenkel verbindenden Steg 53, wobei die Pelotte 50 auch V-förmig ausgebildet sein kann.

In das rückwärtige Bandagenteil 11 der Bandage 10 ist eine zweite oder eine zweite und eine dritte, den Ansatz der ischiokruralen Muskulatur beaufschlagende, ellipsenförmige Pelotte 60, 60' eingesetzt, die ebenfalls aus einem weichen oder weich-elastischen Material besteht.

Fig. 1 und 2 zeigen eine Bandage 10, die als Genu-Kniegelenkbandage 10a ausgebildet ist.

Als weitere Ausführungsform der Bandage 10 ist nach Fig.3 und 4 eine auf der Genu-Kniegelenkbandage aufbauende Patella-Kniegelenkbandage 10b vorgesehen, die sich von der Genu-Kniegelenkbandage 10a nur darin unterscheidet, daß zusätzlich in dem vorderen Bandagenteil 12 mindestens eine, die Pelotte 50 stabilisierende, in Bandagenlängsrichtung verlaufende elastische Spange 70 angeordnet ist, wobei diese Spange 70 integrierter Bestandteil der Pelotte 50 oder seitlich zu dieser liegend ist. Diese Spange 70 weist bevorzugterweise keine Längselastizität auf.

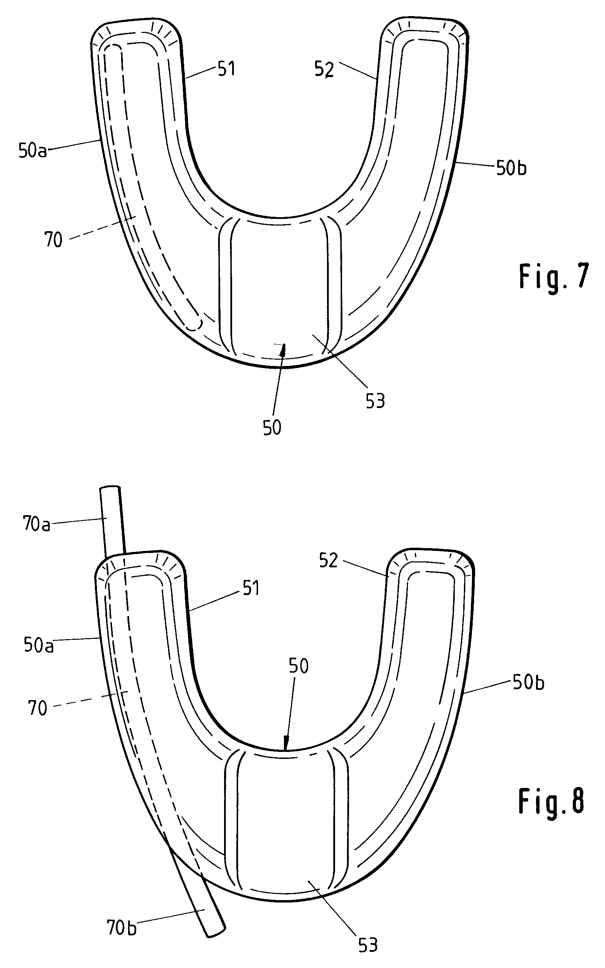

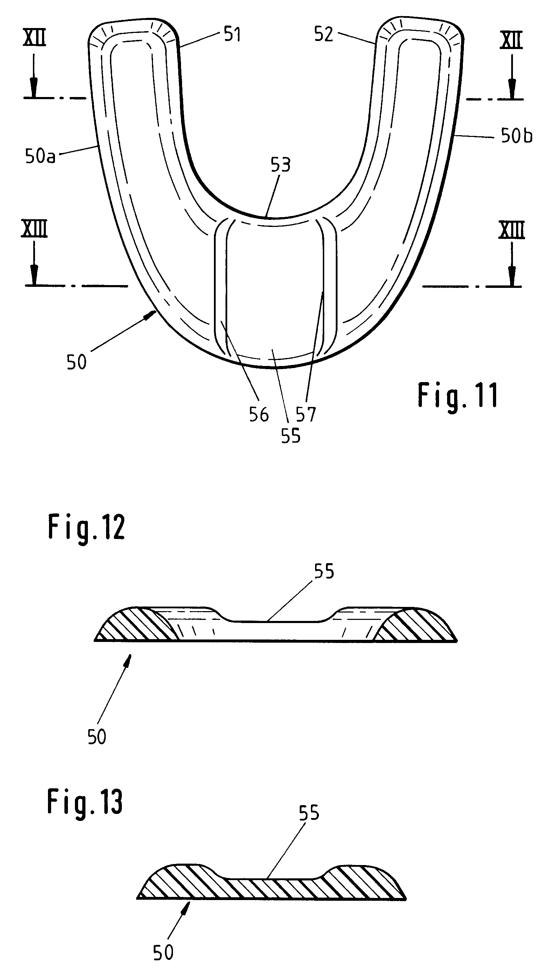

Die Pelotte 50 in dem vorderen Bandagenteil 12 weist bevorzugterweise eine hufeisenförmige Ausgestaltung mit einem bogenförmigen Verlauf der Außenränder 50a,50b der beiden Pelottenschenkel 51,52 auf (Fig.7,10 und 11). Die Pelotte 50 weist eine außenseitig liegende, ebene Grundfläche 54 auf; der Querschnitt der Pelottenschenkel 51,52 und der Querschnitt in einem Teilbereich des Pelottensteges 53 entspricht einem Halbkreis oder einem Teilkreis (Fig.12 und 13). In dem die Pelottenschenkel 51,52 miteinander verbindenden Steg 53 ist mittig eine innenseitig liegende Vertiefung 55 zur Anpassung an den Schienbeinkopf und an den Verlauf des Ligamentum patellae versehen. Die etwa rechteckförmige bzw. quadratische Vertiefung 55 weist in Bandagenlängsrichtung verlaufende seitliche Führungen 56,57 auf. Vermittels dieser Vertiefung 55 in der Pelotte 50 wird ein erhöhter Andruck auf das Kniescheibenband vermieden, wobei diese Profilierung der Pelotte 50 gleichzeitig eine Oberflächenmassage bewirkt. Die seitlichen Führungen 56,57 dienen zur Führung des Kniescheibenbandes. Wesentlich ist jedoch, daß diese Vertiefung 55 in der Pelotte 50 nicht nur zu einer Druckminderung führt, sondern auch der Kontur der Sehne angepaßt ist.

Die Pelotte 50 besteht aus Filz, Moosgummi, Neopren, Gummi, aus einem viskoelastischen Silikonkautschuk oder einem elastischen, kompressiblen, durch Druck verformbaren Silikonkautschuk oder einem Material mit gleichen Elastizitätseigenschaften, wie Naturkautschuk, Silikonschaum od.dgl..

Die Pelotte 50 kann auch beutelförmig ausgebildet sein und eine Füllung aus einem gasförmigen oder flüssigen Medium aufweisen.

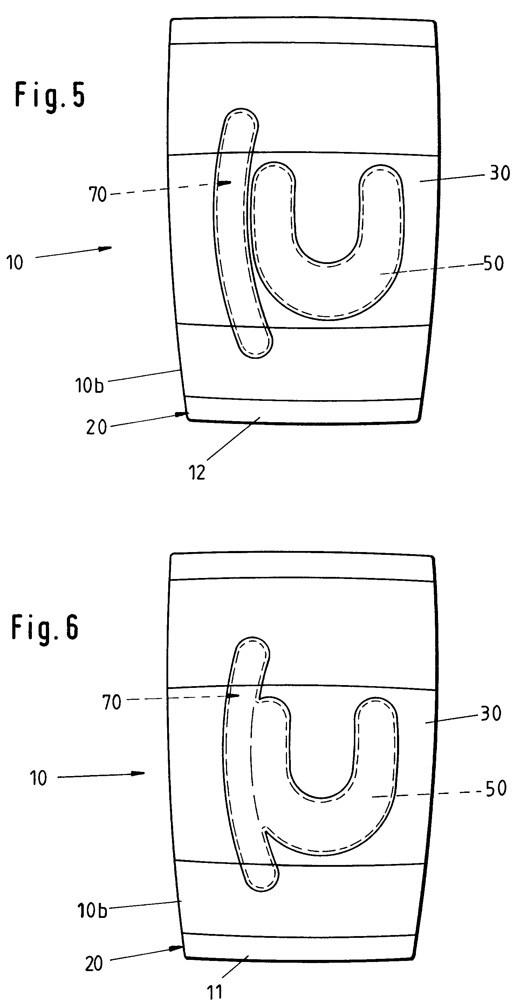

Die Pelottenstabilisierungsspange 70 ist benachbart zu einem der beiden Schenkel 51,52 der Pelotte 50 liegend. Die Spange 70 entspricht in ihrer Formgebung der Form des äußeren Schenkelrandes 50a bzw. 50b der Pelotte 50 (Fig.3). Die Spange 70 kann in einem Abstand von dem Schenkel 51 der Pelotte 50 angeordnet sein. Zwischen dem Schenkel 51 der Pelotte 50 und der Spange 70 ist dann ein Gewebe- oder Gestrickabschnitt 41 angeordnet (Fig.3). Es besteht jedoch auch die Möglichkeit, die Spange 70 abstandslos neben dem Schenkel 51 der Pelotte 50 anzuordnen (Fig.5). Bei einer weiteren Ausführungsform ist die Spange 70 an dem Schenkel 51 der Pelotte 50 angeformt und somit Bestandteil der Pelotte 50 (Fig.6). Die Spange 70 und die Pelotte 50 sind dann einstückig ausgebildet, wobei die Spange 70 eine ihrer Wirkung entsprechende Formelastizität aufweist.

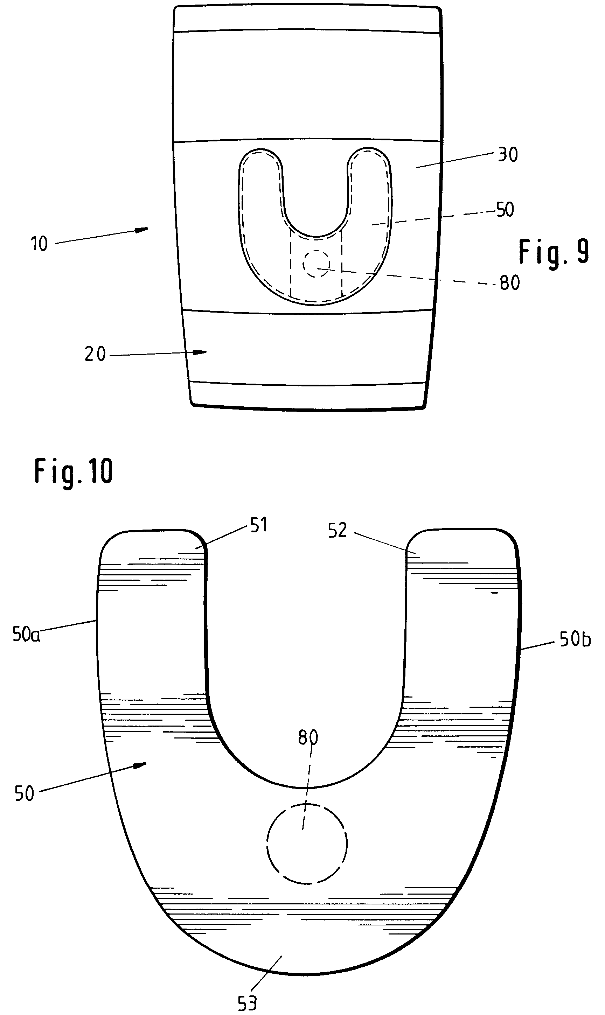

Die Spange 70 ist nach Fig.7 durch den Schenkel 51 der Pelotte 50 hindurchgeführt und in das Pelottenmaterial eingebettet. Bei der in Fig. 8 gezeigten Ausführungsform ist die Spange 70 in den Schenkel 51 der Pelotte 50 eingebettet, und zwar derart, daß die Spange 70 beidendseitig aus dem Pelottenschenkel 51 mit je einem Abschnitt 70a,70b herausragt.

Die Pelottenstabilisierungsspange 70 besteht bevorzugterweise aus einem federnd-elastischen Material, um sich allen Bewegungsabläufen anpassen zu können. Bevorzugterweise besteht die Spange 70 aus einem Kunststoff mit ausreichender Flexibilität und einem Härtegrad, der höher ist als der Härtegrad des Materials, aus dem die Pelotte 50 besteht. In Längsrichtung ist die Spange 70 unelastisch; sie kann jedoch auch in Längsrichtung elastisch sein.

In die Pelotte 50 ist ein knopfförmiger Friktionskern 80 eingearbeitet, der aus einem Material besteht, das gegenüber dem Härtegrad des Pelottenmaterials einen höheren Härtegrad aufweist. Dieser Friktionskern 80 ist in dem die beiden Schenkel 51,52 verbindenden Steg 53 der Pelotte 50, und zwar mittig , angeordnet (Fig. 9 und 10). Die Differenz zwischen der Härte der Pelotte 50 und der Härte des Friktionskerns 80 beträgt mindestens 10 Shore, bevorzugterweise 20 Shore A. Das Material der Pelotte 50 kann auch eine unterhalb 50 Shore A liegende Härte und das Material des Friktionskerns 80 eine oberhalb 50 Shore A liegende Härte aufweisen. Wesentlich ist, daß der Friktionskern 80 aus einem Material besteht, das gegenüber dem Material der Pelotte 50 etwas härter ist.

Während die Pelotte 50 aus einem weichen oder weich-elastischen Material, bevorzugterweise aus einem viskoelastischen Silikonkautschuk oder einem elastischen,kompressiblen,durch Druck verformbaren Silikonkautschuk, z.B. mit einer Härte von 40 Shore A, einem Silikonkautschuk mit einer Härte von 9 bis 13 Shore A oder einem kompressiblen, durch Druck verformbaren und ohne Sprungelastizität in seine Form sich rückbildenden Silikonkautschuk vom Typ eines nach dem Verfahren der Polyaddition vulkanisierenden Kaltkautschuk, der neben einer hohen Flexibilität eine unter 4 Shore A liegende Härte aufweist, wobei jedoch auch Silikonkautschuke eingesetzt werden können, deren Härte oberhalb von 4 Shore A liegt, besteht, besteht der Friktionskern 80 aus einem harten oder inkompressiblen Kunststoff. Derartige, für die Herstellung der Pelotte 50 verwendete viskoelastische Silikonkautschuke oder Materialien mit gleichen Elastizitätseigenschaften besitzen die Eigenschaft, bei angelegter Bandage mit einer derartigen Pelotte 50 aufgrund der gleitenden Bewegung, ausgelöst durch Masseverschiebung, bei Druckanwendung oder bei Bewegungsabläufen im Auflagebereich massierend zu wirken. Für die Herstellung der Pelotte 50 sollte immer ein Material gewählt werden, welches viskoelastisch ist und aufgrund seiner elastischen Eigenschaften eine Massage bewirkt.

Der Friktionskern 80 besteht gegenüber der Pelotte 50 aus einem harten oder inkompressiblen Kunststoff, mit z.B. einer über 50 Shore A liegenden Härte und weist somit gegenüber der Pelotte 50 eine weitaus größere Härte auf, damit bei einer Bewegung eine gezielte Friktionsmassage auf spezielle Schmerzpunkte des Gelenkes erreicht wird. Als Material für den Friktionskern 80 kommt natürlicher oder künstlicher Kautschuk oder Hartgummi infrage. So kann u.a. ein Chloropren-Polymerisat (Handelsname NEOPREN) mit einer Härte von 50 Shore A , ein gummielastisches, vernetztes Polyurethan (Handelsname VULKOLLAN) mit einer Härte von 65 bis 90 Shore A, ein Silikonkautschuk mit einer Härte von 60 Shore A, ein Ethylen-Propylen-Dien-Kautschuk (EPDM) mit einer Härte von 80 Shore A oder ein Copolymerisat mit Acrylnitril (Handelsname PERBUNAN) mit einer Härte von 70 Shore A, ferner ein Polyamid verwendet werden. Jedoch auch andere Kunststoffe oder Naturstoffabkömmlinge können zur Herstellung des Friktionskerns 80 herangezogen werden. Wesentlich ist, daß der Friktionskern 80 eine ausreichende Härte aufweist, um die gezielte Friktionsmassage auf spezielle Schmerzpunkte ausüben zu können. Der Friktionskern 80 kann auch aus Metall oder Holz bestehen.

Der Friktionskern 80 kann in der Pelotte 50 auswechselbar angeordnet sein. Hierzu ist die Pelotte 50 mit einer in der Zeichnung nicht dargestellten Ausnehmung von etwa der Größe des Friktionskerns 80 versehen, in die der Friktionskern 80 vermittels eines leichten Druckes eingedrückt wird. Die die Ausnehmung in der Pelotte 50 begrenzte Innenwandfläche weist bevorzugterweise eine Profilgebung auf, die einen Eingriff in das Profil der umlaufenden Wandfläche des Friktionskerns 80 ermöglicht und da das Pelottenmaterial elastisch ist, der Friktionskern 80 jedoch gegenüber dem Pelottenmaterial eine größere Härte aufweist, läßt sich der Friktionskern 80 in die Ausnehmung pressen, wobei während des Preßvorganges das Profil der Innenwandfläche so zusammengepreßt wird, daß der Friktionskern 80 gänzlich in die Ausnehmung gleiten kann und aufgrund des Rückstellvermögens des Materials der Pelotte 50 wird das Pelottenmaterial in das Randprofil des Friktionskerns 80 gepreßt, so daß dieser fest in der Pelotte 450 gehalten wird. Durch entsprechende Verformung der Pelotte 50 durch eine starke äußere Druckanwendung kann der Friktionskern 80 aus der Pelotte 50 herausgedrückt werden. Dadurch ist die Möglichkeit gegeben, Friktionskerne unterschiedlicher Härte verwenden zu können. In dem Fall, in dem Pelotten 50 mit auswechselbaren Friktionskernen 80 verwendet werden, ist die Pelotte 80 an dem Schlauchkörper 20 der Bandage 10 derart befestigt, daß ein Abnehmen der Pelotte 50 möglich ist.

Der Friktionskern 80 entsprechend Fig. 28 mit einem kreisförmigen, quadratischen, rechteckförmigen oder eine andere geoemtrische Form aufweisenden Querschnitt weist im Bereich seiner umlaufenden Wandfläche 81 eine Einziehung in Form einer Nut 82, rillenförmige Ausnehmungen, Hinterschneidungen, Verzahnungen od.dgl. auf, die zur Aufnahme des Pelottenmaterials dient, damit der Friktionskern 80 in dem Formkörper der Pelotte 50 in seiner Lage fixiert ist. Bevorzugterweise ist die Anordnung des Friktionskerns 80 in der Pelotte 50 so getroffen, daß dieser dicht unterhalb der Oberfläche der Pelotte 50 zu liegen kommt, um so einen hohen Druck auf das Gelenk ausüben zu können.

Der aus einem harten oder inkompressiblen Material bestehende Friktionskern 80 ist in dem Material der Pelotte 50 in seiner Lage fixiert. Der Friktionskern 80 weist eine kugelkappenförmige Oberfläche 83 auf bzw. eine Oberflächenausgestaltung, die in Ausgestaltung und Formgebung mit der Oberflächengestaltung der Pelotte 50 im Anordnungsbereich des Friktionskernes 80 entspricht.

Nach einer weiteren Ausführungsform ist das Material des aus einem Kunststoff, z.B. Silikonkautschuk, bestehenden Friktionskerns 80 mit dem Material der Pelotte 50 verschmolzen und mit dessen Formkörper unlösbar verbunden. Der Friktionskern 80 kann auch bei der Herstellung der Pelotte 50 durch Materialaushärtung eines Abschnittes, der den späteren Friktionskern bilden soll und daher eine größere Härte gegenüber dem weichen Material der Pelotte 50 aufweist, der Pelotte 50 erhalten werden. In beiden Fällen sollte als Material bevorzugterweise Silikonkautschuk verwendet werden.

Die Pelotte 50 kann auch beutelförmig ausgebildet sein.

Dieser Beutel besteht dann aus weich-elastischen Kunststoffen. Der Innenraum des Beutels ist mit einem gasförmigen Medium, wie z.B. Luft, oder einem flüssigen Medium, wie z.B. einem zähflüssigen Silikonöl, Wasser od.dgl. gefüllt. Der Friktionskern 80 ist dann an der Innenwandfläche des Beutels in seiner Lage fixiert.

Die Genu-Kniegelenkbandage 10a ist mit einem in ihrer Pelotte 50 angeordneten, wie oben beschriebenen Friktionskern 80 versehen (Fig. 1 und 2), wohingegen die Patella-Kniegelenkbandage 10b mit der Pelotte 50 versehen ist, die mit oder ohne Friktionskern 80 ausgebildet sein kann (Fig.3 und 4). Die Wirkungsweise der Pelotte 50 ist die einer Druckpelotte.

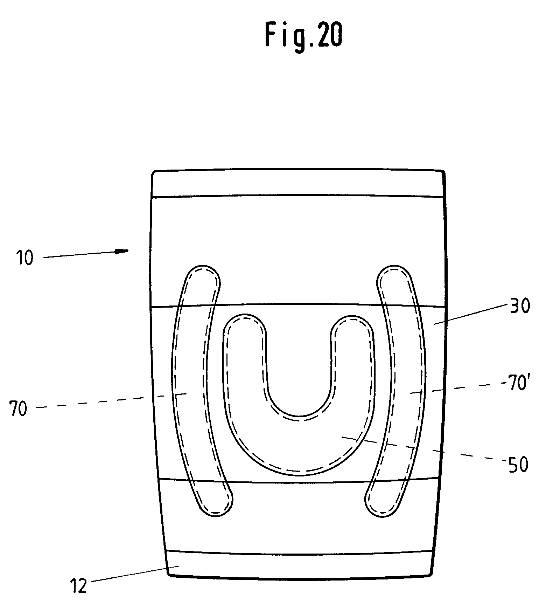

Bei dem in Fig. 3 und 4 gezeigten Ausführungsbeispiel ist seitlich der Pelotte 50 eine Pelottenstabilisierungsspange 70 angeordnet. Es besteht jedoch auch die Möglichkeit, in dem vorderen Bandagenteil 12 zu beiden Seiten der Schenkel 51,52 der Pelotte 50 je eine Pelottenstabilisierungsspange 70,70' anzuordnen (Fig.20).

Die nach einer weiteren Ausführungsform im rückwärtigen Bandagenteil 11 eingenähten Pelotten 60,60' bestehen aus dem gleichen Material wie die Pelotte 50. Neben einer ellipsenförmigen Ausgestaltung können die Pelotten 60,60' auch eine andere Formgebung aufweisen. Jede dieser beiden Pelotten 60, 60' ist als wulstförmiger Körper ausgebildet. Anstelle von zwei Pelotten 60,60' kann in das rückwärtige Bandagenteil 11 auch nur eine Pelotte 60 bzw. 60' eingenäht sein.

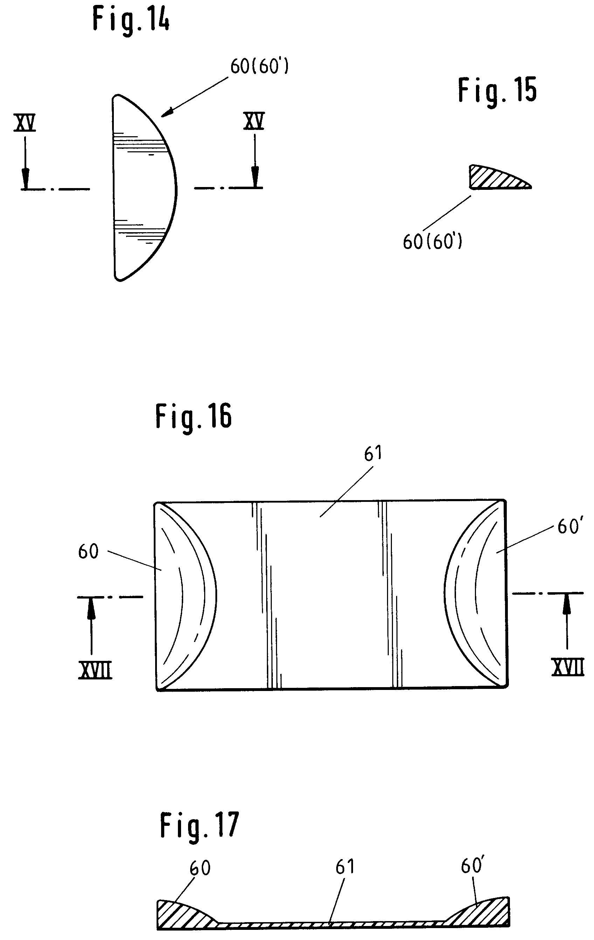

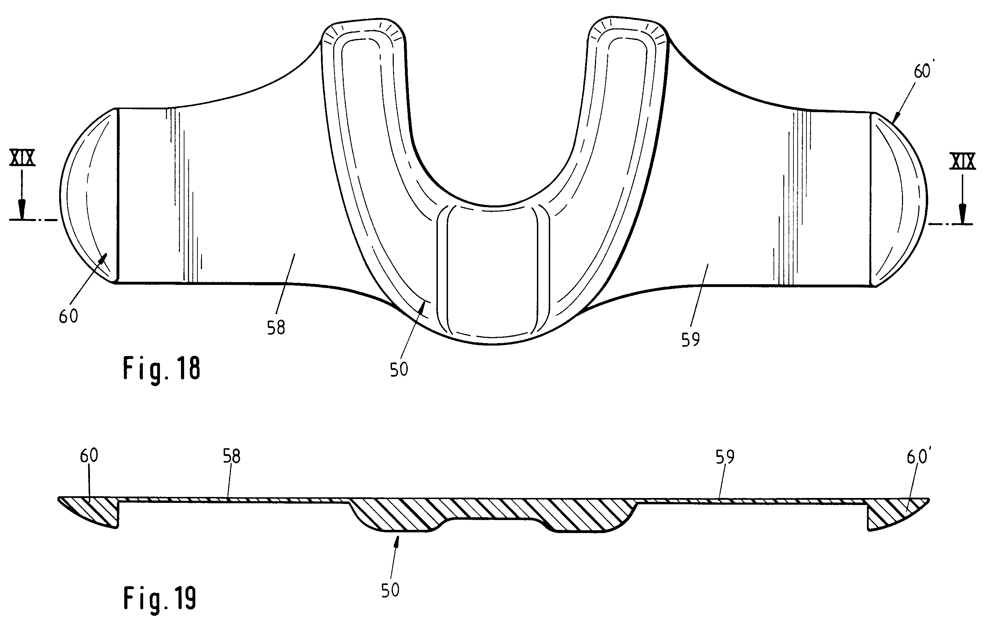

Die beiden Pelotten 60,60' in dem rückwärtigen Bandagenteil 11 können über einen Steg 61 miteinander verbunden sein (Fig.16 und 17). Es besteht darüber hinaus auch die Möglichkeit, die beiden Pelotten 60,60' in dem rückwärtigen Bandagenteil 11 über Stege 58,59 mit den Schenkeln 51,52 der Pelotte 50 zu verbinden (Fig. 18 und 19). Die die Pelotten 60,60' miteinander oder mit der Pelotte 50 verbindenden Stege 61 und 58,59 sind dünnwandig ausgebildet und bestehen aus dem gleichen Material wie die Pelotten 50, 60, 60', so daß diese Stege 61 und 58,59 eine hohe Flexibilität besitzen.

Das rückwärtige Bandagenteil 11 ist mit dem vorgespannten Wellengestrick 40 eingenäht (Fig.1 und 2). Die Pelotte 50 sowie die Pelotten 60,60' und die Pelottenstabilisierungsspange 70 sind im Bereich des in den Schlauchkörper 20 der Bandage 10 integrierten Einsatzes 30 aus dem Wellengestrick 40 angeordnet.

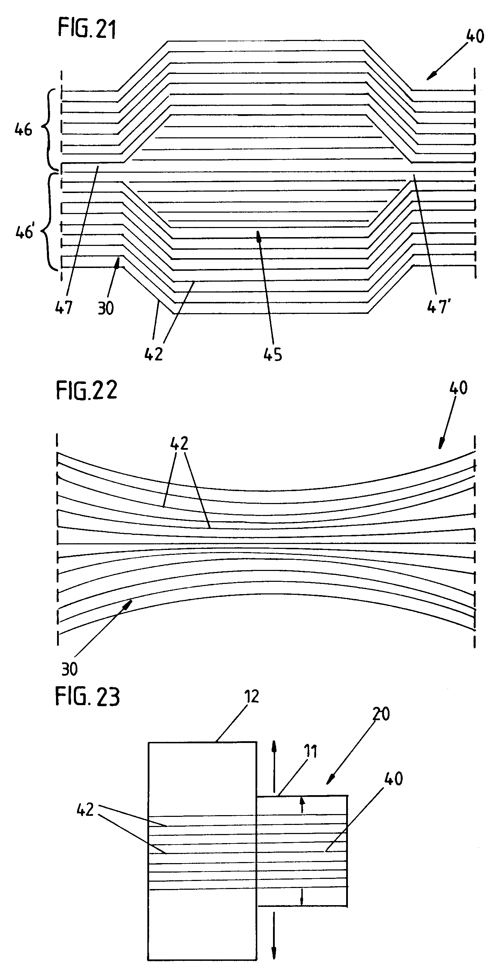

Das Wellengestrick 40 des Einsatzes 30 weist im vorderen Bandagenteil 12 eine größere Anzahl von Wellen 42 als im rückseitigen Bandagenteil 11 auf. Die Führung der Wellen 42 des Wellengestrickes 40 des Einsatzes 30 geht von einer geringeren Anzahl von Wellen im rückwärtigen Bandagenteil 11 aus zu einer Teilung der Wellen 42 in zwei im Abstand voneinander verlaufende Wellengruppen 46,46' in dem vorderen Bandagenteil 12, wobei in dem zwischen den beiden Wellengruppen 46,46' in dem vorderen Bandagenteil 12 ausgebildeten Zwischenraum 45 eine in keilförmige Endabschnitte 47,47' auslaufende Wellengruppe 46'' angeordnet ist (Fig. 21 und 22). Beim Zusammennähen des vorderen Bandagenteils 12 mit dem rückwärtigen Bandagenteil 11 wird das Wellengestrick 40 mit der geringeren Anzahl an Wellen 42 bei einem gleichzeitigen Zusammenziehen des Wellengestricks in dem vorderen Bandagenteil 12 gedehnt (Fig. 23). Eine Besonderheit der Bandage 10 besteht darin, daß für den Einsatz 30, der dort vorgesehen ist, wo die höchsten Dehnungs-Wechselbeanspruchungen der Bandage auftreten, eine besondere Textilstruktur, nämlich das Wellengestrick 40, verwendet wird, welches nachstehend näher erläutert wird.

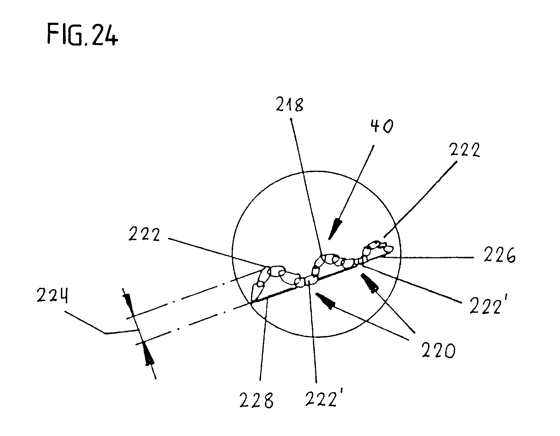

Die streifenförmige Wiedergabe des Einsatzes 30 in den Fig. 21 und 22 deutet an, daß das Wellengestrick 40 in diesem Bereich ein Relief bildet, welches in der Darstellung gemäß Fig.24 verdeutlicht wird. Bei diesem Relief handelt es sich um eine Wellenstruktur, die zumindest auf einer Seite der Bandage - bei der in den Zeichnungen gezeigten Ausführungsformen auf der dem Körper abgewandten Seite - ausgebildet wird. Dabei ist eine Reihe von Halbwellen 218 unter Zwischenschaltung von Knotenpunkten bzw. Knotenbereichen 220 aneinandergereiht, die wie folgt entstehen:
Ein von Maschen 222 gebildetes Deckgestrick 224 ist im Bereich einzelner Maschen 222' mit einer elastischen Fadenanordnung 226 auf der Unterseite des Deckgestricks 224 derart fest verbunden, daß eine Reihe von Maschen 222 des Deckgestricks 224 - bei der Ausführungsform vier - von einer längeren Masche 228 der darunterliegenden, elastischen Fadenanordnung 226 überbrückt wird. Die Maschen 222 zwischen den Knotenpunkten bzw. -bereichen 220 werden dadurch nach oben ausgebaucht, wodurch die Querwellen 218 vorgespannt und stabilisiert werden. Durch Variation der Anzahl der überbrückten Maschen 222 und/oder der Maschen 228 kann auf das Verformungsverhalten und die Dauerelastizität gezielt Einfluß genommen werden.

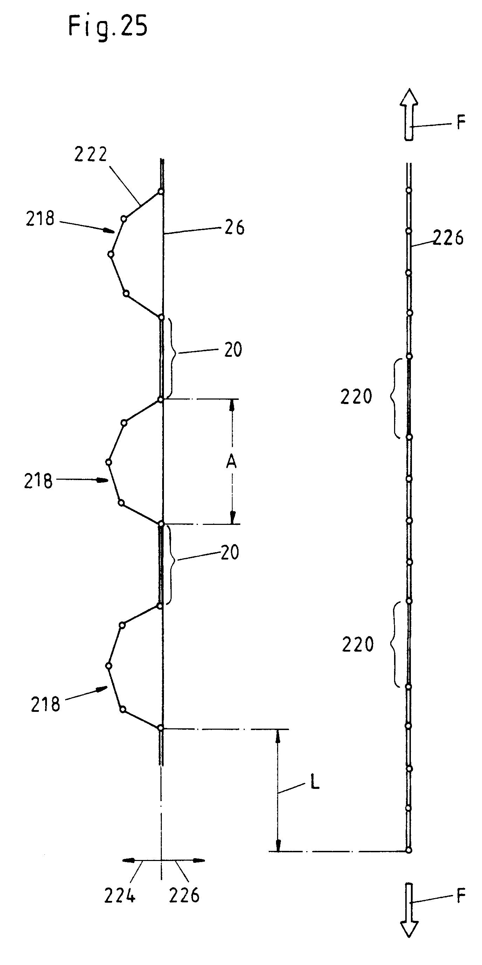

Das Verformungsverhalten des auf diese Art und Weise gebildeten Wellengestricks 40 geht im einzelnen aus der Darstellung gemäß Fig.25 hervor. Auf der linken Seite ist das Wellengestrick im entlasteten Zustand gezeigt. Die Maschen sind durch Linien angedeutet und die Verbindung der Maschen untereinander durch kleine Kreise.

Das auf der Unterseite vorgesehene elastische Garn 226 überbrückt die Knotenbereiche 220, zwischen denen jeweils vier Maschen 222 des Deckgestricks 224 ausgebildet sind.

Durch die Vorspannung der elastischen Fadenanordnung 226 werden die Maschen 222 zu Halbwellen 218 ausgebaucht, wodurch die Reliefstruktur entsteht. Die Halbwellen 218 weisen jeweils zwischen zwei und zwölf, vorzugsweise vier, Maschenreihen auf.

Auf der rechten Seite ist dargestellt, wie sich das Wellengestrick 40 bei Beanspruchung durch eine Zugkraft F verhält. Es ist erkennbar, daß die elastische Fadenanordnung 226 zwischen den Knotenbereichen 220 gedehnt wird, ohne daß eine zusätzliche Dehnungsbeanspruchung im Bereich der Halbwellen 218, d.h. im Bereich der Maschen 222, auftritt. Das Wellengestrick 40 kann sich dementsprechend um das Maß L längen, bevor die Maschen 222 des Deckgestricks 224 auf Zug beansprucht werden. Dieses Maß L schafft dementsprechend eine Dehnungsreserve des Bandagengestricks im Vergleich zu herkömmlichen textilen Flächengebilden.

Die Verbindung zwischen Deckgestrick 224 und elastischer Fadenanordnung 226 kann auf verschiedenste Art und Weise erfolgen. Es ist auch möglich, die Verbindung derart zu wählen bzw. herzustellen, daß auf beiden Seiten des Wellengestricks 40 Wellen 42 gebildet werden. Das Deckgestrick 224 braucht nicht einflächig ausgebildet zu sein.

Durch die vorstehende Struktur des Wellengestricks 40 kann trotz Bereitstellung einer hohen Dehnungselastizität für das Deckgestrick normales Strickgarn, wie z.B. Baumwolle oder Polyamidgarn, verwendet werden. Für die elastische Fadenanordnung 226 wird vorzugsweise höherelastisches Garn, wie z.b. Umwindungsgarn, verwendet, wobei dieser elastische Faden zusätzlich plattiert werden kann, um die Verschleißfestigkeit des textilen Gewebes bzw. Gewirks zu verbessern.

In die Maschen 222 des Deckgestricks 224 kann ferner ein Einlegefaden eingearbeitet werden, um auch in diesem Bereich der wellenförmigen Reliefstruktur eine Kompressionswirkung der Bandage zu erreichen.

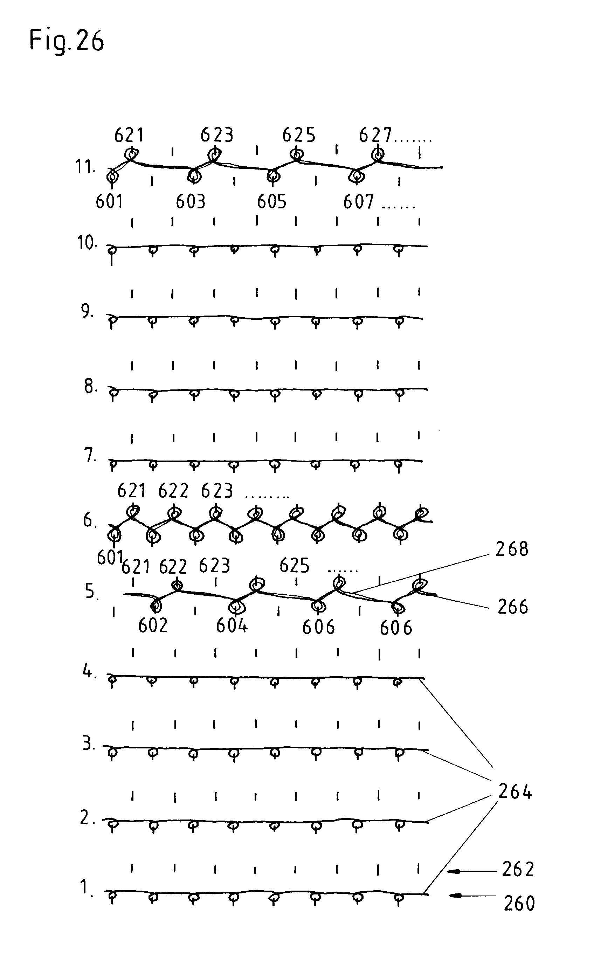

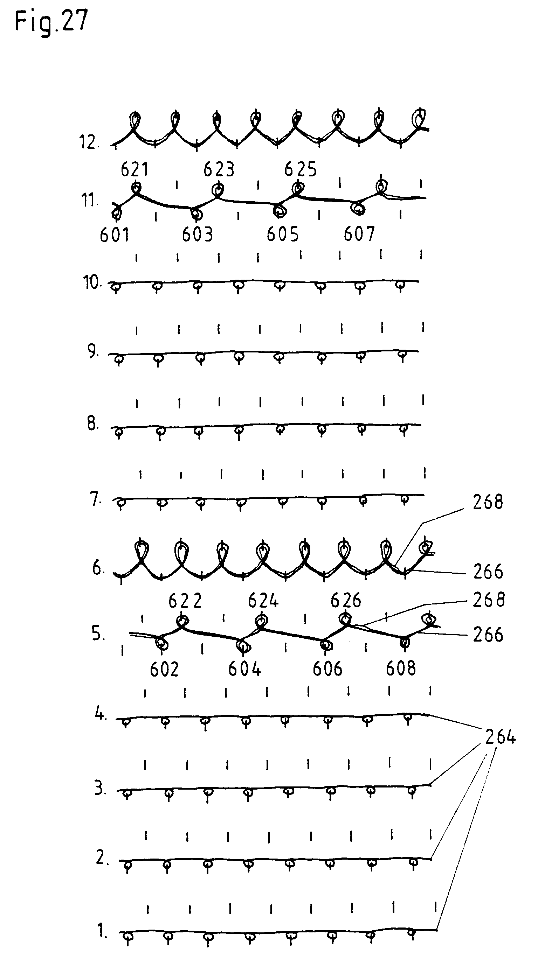

In den Fig. 26 und 27 sind zwei Möglichkeiten aufgezeigt, wie das vorstehend erläuterte Wellengestrick 40 auf automatischen Strickmaschinen hergestellt werden kann. Hierbei werden zwei Nadelbetten verwendet, nämlich ein erstes Nadelbett 260 und ein zweites Nadelbett 262 mit im gleichen Abstand zueinander angeordneten Nadeln. Gestrickt werden auf dem ersten Nadelbett 260 mehrere - im gezeigten Ausführungsbeispiel vier - Maschenreihen aus normalem Strickgarn, wie z.B. Baumwoll- oder Polyamidgarn 264. Anschließend werden zwei Maschenreihen auf beiden Nadelbetten 260,262 mit elastischem Garn, wie z.B. Gummi oder Umwindungsgarn 268 gestrickt, wobei diesem elastischen Garn vorzugsweise ein Plattierfaden 266 beigegeben wird. Bei der fünften Strickreihe wird nur auf ausgewählten Nadeln 602,604,606,... usw. bzw. 622,624,626,... usw. beider Nadelbetten gestrickt. Der Plattierfaden kann von einem Polyamid HE-Faden gebildet sein. Die Darstellung läßt erkennen, daß die Maschenreihe sechs wieder auf allen Nadeln der beiden Nadelbetten 260,262 gestrickt wird. Dies ist jedoch nicht unbedingt erforderlich.

Es folgen dann wiederum vier Maschenreihen mit gewöhnlichem Garn auf dem ersten Nadelbett 260 und abschließend zwei weitere Maschenreihen unter Verwendung elastischen Garns, wobei sich die elfte Strickreihe von der fünften Strickreihe dadurch unterscheidet, daß die beteiligten Nadeln der Nadelbetten 260,262 um eines versetzt sind.

Fig. 27 zeigt ein anderes Muster mit einer etwas anderen Bindung im Bereich der späteren Gestrick-Knoten 220. Die Strickreihen eins bis fünf und sieben bis elf entsprechen dem Strickmuster gemäß Fig.26. Unterschiedlich ist lediglich die Ausbildung der 6. und der 12 Strickreihe, so daß ein näheres Eingehen auf diese Figur nicht erforderlich erscheint.

Abweichend von dem zuvor beschriebenen Herstellungsverfahren ist es auch möglich, zunächst auf einem Nadelbett eine oder mehrere Maschenreihen aus elastischem Garn, wie z.B. Umwindegarn, zu stricken, und auf dem anderen Nadelbett Maschenreihen aus normalem Strickgarn, wie z.B. Baumwoll- oder Polyamidgarn, wobei anschließend eine oder mehrere Maschenreihen mit allen oder einzelnen Nadeln beider Nadelbetten gestrickt werden.

Es erfolgt keine Beschränkung darauf, daß der Einsatz 30 aus einem vorangehend beschriebenen Wellengestrick 40 besteht. Entscheidend ist lediglich, daß das den Einsatz 30 bildende textile Flächengebilde einen solchen Aufbau erhält, daß eine entweder eingearbeitete oder nach vorbestimmtem Muster mit einer textilen Deckstruktur verbundene elastische Faden Anordnung dem Flächengebilde eine solche innere Vorspannung gibt, daß zumindest auf einer Seite ein wellenartiges Relief entsteht, das dann durch äußere Beanspruchung zunächst glatt bzw. glatter gezogen werden kann, ohne bereits in dieser Phase die Deckstruktur auf Dehnung zu beanspruchen.

Der Einsatz 30 aus dem Wellengestrick 40 verhindert eine Faltenbildung bzw. wirkt einer Faltenbildung entgegen, insbesondere im inneren Beugebereich der angelegten Bandage 10.

Bevorzugterweise ist die Pelottenstabilisierungsspange 70 beidendseitig aus dem Einsatz 30 des Wellengestricks 40 herausgeführt, wobei die freien Spangenenden in das Gewebe oder Gewirk des Schlauchskörpers 20 der Bandage 10 eingenäht sind.

Um vom Stegbereich der Pelotte 50 einen fast stufenlosen Übergang zur Bandage zu erhalten, kann der Steg der Pelotte 50 mit einer zungenartigen, zum äußeren Rand verjüngend auslaufenden Anformung versehen sein, die aus dem Pelottenmaterial besteht. Auch besteht die Möglichkeit, den äußeren Randbereich des Steges in seinen zu beiden Seiten der Stegmitte liegenden Stegabschnitten verjüngend auslaufen zu lassen. Der äußere Rand des Pelottensteges ist somit nach außen flach auslaufend ausgebildet. Die freien Enden der beiden Schenkel der Pelotte können elefantenfußartig verbreitert ausgebildet sein, wodurch der Auflage- und Wirkungsbereich der Pelotte 50 verbessert wird.

## Patentansprüche

1. Bandage für das Kniegelenk aus elastischem Bandagenstoff in Schlauchform mit einem vorderen Bandagenteil (12) und einem rückwärtigen Bandagenteil (11) und mit gegebenenfalls im rückwärtigen Bandagenteil (11) eingearbeiteten, längsverlaufenden Federstäben, dadurch gekennzeichnet, daß die Bandage (10a) aus einem anatomisch geformten Schlauchkörper (20) aus einem Gewebe oder Gestrick mit einem rundumlaufenden Einsatz (30) aus einem hochelastischen Wellengestrick (40) besteht und daß in dem vorderen Bandagenteil (12) eine im Bereich über der Kniescheibe bei angelegter Bandage (10a) liegende erste, nach oben offene und die Quadrizeps-Sehne freilassende Pelotte (50) aus einem weichen oder weichelastischen Material angeordnet ist.

2. Bandage für das Kniegelenk aus einem elastischen Bandagenstoff in Schlauchform mit einem vorderen Bandagenteil (12) und einem rückwärtigen Bandagenteil (11) und mit gegebenenfalls im rückwärtigen Bandagenteil eingearbeiteten, längsverlaufenden Federstäben, dadurch gekennzeichnet, daß die Bandage (10b) aus einem anatomisch geformten Schlauchkörper (20) aus einem Gewebe oder Gestrick mit einem rundumlaufenden Einsatz (30) aus einem hochelastischen Wellengestrick (40) besteht, und daß in dem vorderen Bandagenteil (12) eine im Bereich über der Kniescheibe bei angelegter Bandage (10b) liegende erste, nach oben offene und die Quadrizeps-Sehne freilassende Pelotte (50) aus einem weichen oder weichelastischen Material und mindestens eine, die erste Pelotte (50) stabilisierende, in Bandagenlängsrichtung verlaufende Spange (70) angeordnet sind, wobei die Spange (70) integrierter Bestandteil der ersten Pelotte (50) oder seitlich zu dieser liegend ist.

3. Bandage für das Kniegelenk aus elastischem Bandagenstoff in Schlauchform mit einem vorderen Bandagenteil (12) und einem rückwärtigen Bandagenteil (11) und mit gegebenenfalls im rückwärtigen Bandagenteil (11) eingearbeiteten, längsverlaufenden Federstäben, dadurch gekennzeichnet, daß die Bandage (10a) aus einem anatomisch geformten Schlauchkörper (20) aus einem Gewebe oder Gestrick mit einem rundumlaufenden Einsatz (30) aus einem hochelastischen Wellengestrick (40) besteht, daß in dem vorderen Bandagenteil (12) eine im Bereich über der Kniescheibe bei angelegter Bandage (10a) liegende erste, nach oben offene und die Quadrizeps-Sehne freilassende Pelotte (50) aus einem weichen oder weichelastischen Material angeordnet ist, und daß in das rückwärtige Bandagenteil (11) eine zweite oder eine zweite und eine dritte, den Ansatz der ischiokruralen Muskulatur beaufschlagende Pelotte (60,60') aus einem weichen oder weich-elastischen Material eingesetzt sind.

4. Bandage für das Kniegelenk aus einem elastischen Bandagenstoff in Schlauchform mit einem vorderen Bandagenteil (12) und einem rückwärtigen Bandagenteil (11) und mit gegebenenfalls im rückwärtigen Bandagenteil eingearbeiteten, längsverlaufenden Federstäben, dadurch gekennzeichnet, daß die Bandage (10b) aus einem anatomisch geformten Schlauchkörper (20) aus einem Gewebe oder Gestrick mit einem rundumlaufenden Einsatz (30) aus einem hochelastischen Wellengestrick (40) besteht, daß in dem vorderen Bandagenteil (12) eine im Bereich über der Kniescheibe bei angelegter Bandage (10b) liegende erste, nach oben offene und die Quadrizeps-Sehne freilassende Pelotte (50) aus einem weichen oder weichelastischen Material und mindestens eine, die erste Pelotte (50) stabilisierende, in Bandagenlängsrichtung verlaufende Spange (70) angeordnet sind, wobei die Spange (70) integrierter Bestandteil der ersten Pelotte (50) oder seitlich zu dieser liegend ist, und daß in das rückwärtige Bandagenteil (11) eine zweite oder eine zweite und eine dritte, den Ansatz der ischiokruralen Muskulatur beaufschlagende Pelotte (60,60') aus einem weichen oder weich-elastischen Material eingesetzt sind.

5. Bandage nach einem der Ansprüche 1 bis 4, dadurch gekennzeichnet, daß die erste Pelotte (50) U-förmig oder V-förmig ausgebildet ist und bevorzugterweise eine hufeisenförmige Ausgestaltung mit einem bogenförmigen Verlauf der Außenräder (50a,50b) der beiden Pelottenschenkel (51,52) aufweist.

6. Bandage nach einem der Ansprüche 1 bis 5, dadurch gekennzeichnet, daß die erste Pelotte (50) eine außenseitig liegende, ebene Grundfläche (54), einen halbkreis- oder teilkreisförmigen Schenkelquerschnitt und in dem die Pelottenschenkel (51,52) miteinander verbindenden Steg (53) mittig eine innenseitig liegende Vertiefung (55) mit in Bandagenlängsrichtung verlaufenden seitlichen Führungen (56,57) zur Anpassung an den Schienbeinkopf und an den Verlauf des Ligamentum patellae aufweist.

7. Bandage nach einem der Ansprüche 2 und 4, dadurch gekennzeichnet, daß die Pelottenstabilisierungsspange (70) benachbart zu einem der beiden Pelottenschenkel (51,52) der ersten Pelotte (50) liegend ist und eine der Form des äußeren Schenkelrandes (50a;50b) entsprechende Form aufweist.

8. Bandage nach einem der Ansprüche 2 ,4 bis 7, dadurch gekennzeichnet, daß die Pelottenstabilisierungsspange (70) in einem Abstand von dem Pelottenschenkel (51) der beiden Pelottenschenkel (51,52) der ersten Pelotte (50) angeordnet ist.

9. Bandage nach Anspruch 8, dadurch gekennzeichnet, daß zwischen dem Pelottenschenkel (51) der ersten Pelotte (50) und der Pelottenstabilisierungsspange (70) ein Gewebe- oder Gestrickabschnitt (41) angeordnet ist.

10. Bandage nach einem der Ansprüche 2 ,4 bis 9, dadurch gekennzeichnet, daß die Pelottenstabilisierungsspange (70) abstandslos neben dem Pelottenschenkel (51) der beiden Pelottenschenkel (51,52) der ersten Pelotte (50) angeordnet ist.

11. Bandage nach einem der Ansprüche 2,4 bis 9, dadurch gekennzeichnet, daß die Pelottenstabilisierungsspange (70) an dem Pelottenschenkel (51) der beiden Pelottenschenkel (51,52) der ersten Pelotte (50) angeformt ist.

12. Bandage nach einem der Ansprüche 2,4 bis 9, dadurch gekennzeichnet, daß die Pelottenstabilisierungsspange (70) durch einen der beiden Pelottenschenkel (51,52) der ersten Pelotte (50) hindurchgeführt und in das Pelottenmaterial eingebettet ist.

13. Bandage nach einem der Ansprüche 2,4 bis 9, dadurch gekennzeichnet, daß die Pelottenstabilisierungsspange (70) durch einen der beiden Pelottenschenkel (51,52) der ersten Pelotte (50) hindurchgeführt und in das Pelottenmaterial eingebettet ist, wobei die Pelottenstabiliserungsspange (70) beidendseitig aus dem Pelottenschenkel (51) mit einem Abschnitt (70a;70b) herausgeführt ist.

14. Bandage nach einem der Ansprüche 1 bis 13, dadurch gekennzeichnet, daß die erste Pelotte (50) einen knopfförmigen Friktionskern (80) enthält, der in das Pelottenmaterial eingearbeitet ist und der aus einem Material besteht, das gegenüber dem Härtegrad des Pelottenmaterials einen höheren Härtegrad aufweist.

15. Bandage nach Anspruch 14, dadurch gekennzeichnet, daß der Friktionskern (80) in dem die Pelottenschenkel (51,52) der ersten Pelotte (50) verbindenden Steg (53) mittig in diesem angeordnet ist.

16. Bandage nach einem der Ansprüche 14 und 15, dadurch gekennzeichnet, daß die Differenz zwischen der Härte der ersten Pelotte (50) und der Härte des Friktionskerns (80) mindestens 10 Shore A, vorzugsweise 20 Shore A, beträgt.

17. Bandage nach einem der Ansprüche 14 bis 16, dadurch gekennzeichnet, daß das Material der ersten Pelotte (50) eine unterhalb 50 Shore A liegende Härte und das Material des Friktionskerns (80) eine oberhalb 50 Shore A liegende Härte aufweist.

18. Bandage nach einem der Ansprüche 14 bis 17, dadurch gekennzeichnet, daß der Friktionskern (80) zur Lagenfixierung in der ersten Pelotte (50) im Bereich seiner umlaufenden Wandfläche (81) eine rillenförmige Ausnehmung, Einziehung, wie Nut, Hinterschneidung, Verzahnung od.dgl., (82) zur Aufnahme von Pelottenmaterial aufweist.

19. Bandage nach einem der Ansprüche 14 bis 18, dadurch gekennzeichnet, daß das Material des Friktionskerns (80) mit dem Material der ersten Pelotte (50) verschmolzen und mit dem Pelottenmaterial unlösbarverbunden ist, wobei der Friktionskern (80) und die erste Pelotte (50) aus Kunststoffen, insbesondere Silikonkautschuken od.dgl., besteht.

20. Bandage nach einem der Ansprüche 14 bis 19, dadurch gekennzeichnet, daß der Friktionskern (80) bei der Herstellung der ersten Pelotte (50) durch Materialaushärtung eines Abschnittes der ersten Pelotte (50) erhalten wird, wobei der Friktionskern (80) und die erste Pelotte (50) aus Kunststoffen, insbesondere Silikonkautschuken od.dgl., unterschiedlicher Härtegrade besteht.

21. Bandage nach einem der Ansprüche 1 bis 20, dadurch gekennzeichnet, daß alle Pelotten (50;60,60') aus Filz, Moosgummi, Neopren, Gummi, einem viskoelastischen Silikonkautschuk oder einem elastischen, kompressiblen, durch Druck verformbaren Silikonkautschuk oder einem Material mit gleichen Elastizitätseigenschaften, wie Naturkautschuk, Silikonkautsachuk od. dgl., besteht.

22. Bandage nach einem der Ansprüche 1 bis 20, dadurch gekennzeichnet, daß eine der Pelotten (50;60,60') oder alle Pelotten (50;60,60') beutelförmig ausgebildet sind und eine Füllung aus einem gasförmigen oder flüssigen Medium aufweisen.

23. Bandage nach einem der Ansprüche 1 bis 21, dadurch gekennzeichnet, daß bei einer beutelförmigen Ausbildung der ersten Pelotte (50) der Friktionskern (80) an der Innenwandfläche des Beutels in seiner Lage fixiert ist.

24. Bandage nach einem der Ansprüche 1 bis 20, dadurch gekennzeichnet, daß der Friktionskern (80) aus einem inkompressiblen Kunststoff, wie einem natürlichen oder künstlichen Kautschuk, Hartgummi, Chloropren-Polymerisat, einem gummielastischen, vernetzten Polyurethan, Polyamid , Metall, Holz od.dgl. besteht.

25. Bandage nach einem der Ansprüche 1 bis 24, dadurch gekennzeichnet, daß in der ersten Pelotte (50) eine den Friktionskern (80) aufnehmende Ausnehmung ausgebildet ist.

26. Bandage nach Anspruch 25, dadurch gekennzeichnet, daß der Friktionskern (80) in der Ausnehmung lösbar mittels Preß- oder Klemmsitz gehalten ist.

27. Bandage nach einem der Ansprüche 1 bis 26, dadurch gekennzeichnet, daß die obere Wandfläche des Friktionskerns (80) bogenförmig, halbkreisförmig oder flach mit abgerundeten Ecken ausgebildet ist.

28. Bandage nach einem der Ansprüche 1 bis 27, dadurch gekennzeichnet, daß in dem vorderen Bandagenteil (12) zu beiden Seiten der Pelottenschenkel (51,52) der ersten Pelotte (50) je eine Pelottenstabilisierungsspange (70,70') angeordnet ist.

29. Bandage nach einem der Ansprüche 1 bis 28, dadurch gekennzeichnet, daß die beiden Pelotten (60,60') in dem rückwärtigen Bandagenteil (11) über einen Steg (61) miteinander verbunden sind.

30. Bandage nach einem der Ansprüche 1 bis 28, dadurch gekennzeichnet, daß die beiden Pelotten (60,60') in dem rückwärtigen Bandagenteil (11) über Stege (58,59) mit den Pelottenschenkeln (51,52) der ersten Pelotte (50) verbunden sind.

31. Bandage nach einem der Ansprüche 29 und 30, dadurch gekennzeichnet, daß die die Pelotten (60,60') in dem rückwärtigen Bandagenteil (11) miteinander oder mit der ersten Pelotte (50) verbindenden Stege (61;58,59) aus dem Pelottenmaterial bestehen.

32. Bandage nach einem der Ansprüche 1 bis 31, dadurch gekennzeichnet, daß die Federstäbe (13,14) eine bogenförmige Ausgestaltung aufweisen.

33. Bandage nach einem der Ansprüche 1 bis 32, dadurch gekennzeichnet, daß das rückwärtige Bandagenteil (11) mit vorgespanntem Gestrick oder Wellengestrick (40) eingenäht ist.

34. Bandage nach einem der Ansprüche 1 bis 33, dadurch gekennzeichnet, daß die erste Pelotte (50) die zweite und die dritte Pelotte (60,60') und die Pelottenstabilisierungsspange (70) im Bereich des Einsatzes (30) aus dem Wellengestrick (40) angeordnet sind.

35. Bandage nach einem der Ansprüche 1 bis 34, dadurch gekennzeichnet, daß die erste Pelotte (50), die zweite und dritte Pelotte (60,60') und die Pelottenstabilisierungsspange (70) in taschenförmigen Aufnahmen angeordnet sind, die an der Bandage (10) ausgebildet sind.

36. Bandage nach einem der Ansprüche 1 bis 35, dadurch gekennzeichnet, daß das Wellengestrick (40) des Einsatzes (30) in dem vorderen Bandagenteil (12) eine größere Anzahl von Wellen (42) als in dem rückwärtigen Bandagenteil (11) aufweist.

37. Bandage nach einem der Ansprüche 1 bis 36, dadurch gekennzeichnet, daß die Führung der Wellen (42) des Wellengestricks (40) des Einsatzes (30) von einer geringen Anzahl von Wellen im rückwärtigen Bandagenteil (11) aus zu einer Teilung der Wellen (42) in zwei im Abstand voneinander verlaufende Wellengruppen (46,46') in dem vorderen Bandagenteil (12) erfolgt, wobei in dem zwischen den beiden Wellengruppen (46,46') eine in keilförmige Endabschnitte (47,47') auslaufende Wellengruppe (46'') angeordnet ist.

38. Bandage nach einem der Ansprüche 1 bis 37, dadurch gekennzeichnet, daß beim Zusammennähen des vorderen Bandagenteils (12) mit dem rückwärtigen Bandagenteil (11) das Wellengestrick (40) mit der geringeren Wellenanzahl in dem rückwärtigen Bandagenteil (11) das Wellengestrick in dem vorderen Bandagenteil (12) zusammengezogen wird.

39. Bandage nach einem der Ansprüche 1 bis 38, dadurch gekennzeichnet, daß das Wellengestrick (40) des Einsatzes (30) eine zumindest auf einer Seite ausgebildete, im wesentlichen senkrecht zur Hauptdehnungsrichtung (F) ausgerichtete Wellenstruktur einer Deckstruktur aufweist, wobei die Wellenstruktur (216,218) durch eine maschenreihenweise eingearbeitete und unter dieser Deckstruktur liegende elastische Fadenanordnung (226) elastisch vorgespannt und stabilisiert ist, die in vorbestimmten Abständen (A) mit der Deckstruktur verbunden ist.

40. Bandage nach einem der Ansprüche 1 bis 38, dadurch gekennzeichnet, daß das Wellengestrick (40) derart geformt ist, daß die Anzahl der Querwellen dort am größten ist, wo aufgrund der Gelenkbeugung der größte Dehnungsweg (L) auftritt.

41. Bandage nach einem der Ansprüche 1 bis 39, dadurch gekennzeichnet, daß die Wellenstruktur (216,218) aus Halbwellen (218) besteht, die jeweils zwischen zwei und zwölf, vorzugsweise vier, Maschenreihen aufweisen.

42. Bandage nach einem der Ansprüche 1 bis 41, dadurch gekennzeichnet, daß das Deckgestrick (224) des Wellengestricks (40) aus einer im Vergleich zur eingearbeiteten oder darunterliegenden Fadenstruktur weniger elastischen Maschenware (264) mit unelastischen Fäden besteht.

43. Bandage nach einem der Ansprüche 1 bis 42, dadurch gekennzeichnet, daß das Deckgestrick (224) aus Baumwoll- und/oder Polyamid-Garn besteht.

44. Bandage nach einem der Ansprüche 1 bis 43, dadurch gekennzeichnet, daß die elastische Fadenanordnung (226) aus Umwindungsgarn (268) besteht oder dieses aufweist.

45. Bandage nach Anspruch 44, dadurch gekennzeichnet, daß das Umwindungsgarn (268) plattiert ist.

46. Bandage nach einem der Ansprüche 1 bis 45, dadurch gekennzeichnet, daß in das Deckgestrick (224) ein Einlegefaden eingearbeitet ist.

47. Bandage nach einem der Ansprüche 1 bis 46, dadurch gekennzeichnet, daß in dem durch Gelenkbeugung höchstbeanspruchten Bereich der Bandage (10) mehrere textile Wellengestricke (40) angeordnet sind, wobei die jeweiligen Querwellen-Ausrichtungen im Winkel zueinander stehen.

48. Bandage nach einem der Ansprüche 1 bis 47, dadurch gekennzeichnet, daß über den das rückwärtige Bandagenteil (11) mit dem vorderen Bandagenteil (12) verbindenden Nähten Federstäbe (13,14) in das Schlauchkörpermaterial eingearbeitet sind.

49. Bandage nach einem der Ansprüche 2,4 bis 48, dadurch gekennzeichnet, daß die Pelottenstabilisierungsspange (70) elastisch ausgebildet ist.

50. Bandage nach einem der Ansprüche 2,4 bis 48, dadurch gekennzeichnet, daß die Pelottenstabilisierungsspange (70) elastisch und in Längsrichtung unelastisch ausgebildet ist.

51. Bandage nach einem der Ansprüche 1 bis 50, dadurch gekennzeichnet, daß die Pelotte (60;60') oder die Pelotten (60,60') ellipsenförmig ausgebildet sind oder eine andere geometrische Form aufweisen.

52. Bandage nach einem der Ansprüche 1 bis 51, dadurch gekennzeichnet, daß die Pelotten (60,60') zu einer Pelotte zusammengefaßt sind.
